Europäisches Patentamt

European Patent Office

Office européen des brevets

(11) Numéro de publication: **0 273 823**
A1

(12) **DEMANDE DE BREVET EUROPEEN**

(21) Numéro de dépôt: 87402901.0

(22) Date de dépôt: 18.12.87

(51) Int. Cl.⁴: **B 01 D 13/02**
A 61 K 9/50

(30) Priorité: 18.12.86 FR 8617745

(43) Date de publication de la demande:
06.07.88 Bulletin 88/27

(84) Etats contractants désignés:
AT BE CH DE ES FR GB GR IT LI LU NL SE

(71) Demandeur: MERO ROUSSELOT SATIA, Société anonyme
15, avenue d'Eylau
F-75116 Paris (FR)

(72) Inventeur: David, Jacky
Quartier Peyreras
F-84800 Isle sur Sorgue (FR)

Lefrancois, Claude
Village Franquetot Cretteville
F-50250 La Haye du Puits (FR)

Ridoux, Claude
Chemin du Moulin 1er
F-84800 Isle sur Sorgue (FR)

(74) Mandataire: Gillard, Marie-Louise et al
Cabinet Beau de Loménie 55, Rue d'Amsterdam
F-75008 Paris (FR)

(54) Microcapsules à base de gélatine et de polysaccharide et leur procédé d'obtention.

(57) Microcapsules à base de gélatine et de polysaccharide anionique de faible masse moléculaire pouvant contenir des matières solides ou des liquides, préparées par coacervation.

Les solutions aqueuses des polysaccharides utilisables présentent une faible viscosité, dont la valeur est fonction de la nature du polysaccharide ; pour une concentration de 15 g/l elle est, à 75°C, en général comprise entre 1 et 20 mPa.s.

Ces polysaccharides sont des produits de dépolymérisation des produits naturels : des alginates, des carraghénanes, des pectines et pectates, des carboxyméthylcelluloses, ces carboxyméthylguars et carboxyméthylamidons.

Les microcapsules peuvent être isolées et séchées.

EP 0 273 823 A1

**Description**

## Microcapsules à base de gélatine et de polysaccharide et leur procédé d'obtention.

La présente invention concerne des microcapsules, à base de gélatine et de polysaccharide , qui peuvent contenir des matières solides ou des liquides, ainsi que leur procédé d'obtention par coacervation complexe.

On sait que, lorsque l'on mélange deux solutions aqueuses colloïdales dont l'une contient un polymère anionique et l'autre un polymère cationique, il se forme par dilution ou modification du pH deux phases dont l'une, appelée coacervat, est riche en polymères et appauvrie en eau tandis que l'autre, le surnageant, est de faible concentration en polymères. Lorsque le milieu contient en outre des liquides en émulsion ou des solides en suspension, le coacervat se dépose autour de ces noyaux liquides ou solides pour former une paroi liquide les isolant du milieu ; le durcissement, in situ, de cette paroi liquide, par un moyen choisi en fonction de la nature des polymères, peut permettre d'obtenir des microcapsules stables en suspension.

Ces suspensions de microcapsules sont très utilisées pour préparer, notamment, des papiers autocopiants sans carbone, et on peut citer, par exemple, les microcapsules décrites dans FR-A-2 378 561 qui sont préparées par coacervation dans des conditions très précises d'agitation afin d'obtenir une suspension de microcapsules dont les agrégats sont de diamètre uniforme et faible, entre 3 et 20 microns ; les polymères utilisés sont les hydrocolloïdes naturels et synthétiques et, avantageusement, ce sont la gélatine et la carboxyméthylcellulose ; de préférence la viscosité de cette gélatine est comprise entre 1,5 mPa.s et 3,5 mPa.s, mesurée selon la norme japonaise JIS K6503, et celle de la carboxyméthylcellulose est comprise entre 2 et 500 mPa.s à 25°C en solution à 2 % ; cette dernière valeur de viscosité correspond à une viscosité de 3,6 à 3,8 mPa.s pour une solution à 1,5 % et à 75°C. La gélatine de faible viscosité facilite la définition des dimensions de particules ; elle est utilisée avec une carboxyméthylcellulose dont la viscosité ne dépasse pas 500 mPa.s afin que les solutions concentrées d'hydrocolloïdes, qui doivent être utilisées, ne gélifient pas au refroidissement, et dont la viscosité est supérieure à 2 mPa.s afin d'obtenir des capsules de résistance convenable. En aucun cas ces microcapsules ne sont isolées du milieu dans lequel elles ont été préparées.

Dans l'art antérieur, la plupart du temps, les microcapsules sont préparées à partir d'un polymère cationique qui est de la gélatine, tandis que le polymère anionique peut être un polysaccharide naturel, tel que la gomme arabique, les alginates, les pectines et les pectates ou un polysaccharide rendu hydrosoluble, tel que les carboxyméthylcelluloses, le carboxyméthylguar et les carboxyméthylamidons : le durcissement de la paroi liquide, obtenu par refroidissement du milieu en dessous de la température de gélification des polymères, est en général complété par action d'un agent classique de réticulation, ou tannage, de la gélatine.

On sait d'autre part que les microcapsules à base de gélatine et de gomme arabique ou d'alginates ou de carboxyméthylcelluloses, dont les solutions aqueuses ont une faible viscosité, peuvent être isolées ; mais seules celles à base de gomme arabique donnent des microcapsules sèches pulvérulentes, s'écoulant facilement ; les autres sont difficiles à sécher et surtout perméables, de telle sorte qu'elles libèrent leur contenu plus ou moins rapidement. Les microcapsules à base de gélatine et d'alginates ou de carboxyméthylcelluloses dont les solutions aqueuses ont une viscosité supérieure à 500 mPa.s environ, mesurée à 25°C à la concentration de 2 %, ne peuvent pas être isolées ; elles éclatent au lavage et/ou au séchage, en libérant leur contenu ; cette instabilité limite leurs applications.

Enfin, on n'a pas encore décrit de microcapsules, sèches ou en suspension, préparées par coacervation de gélatine et d'un autre type de polysaccharide naturel bien connu : le carraghénane ; la demanderesse a constaté que, dans ce cas, il n'y a pas de phénomène de coacervation, mais une floculation rapide, complexe, et il se forme un bloc lors de la gélification, sans encapsulation des particules ou des gouttelettes en suspension dans le milieu.

La présente invention permet d'obtenir, par coacervation complexe, des microcapsules isolables, sèches, s'écoulant librement, dont la paroi est à base de gélatine et de polysaccharide autre que la gomme arabique, et cela sans addition d'un adjuvant antiagglomérant, tel qu'un agent tensioactif ou un agent antichoc. Ceci est d'autant plus important que la quantité de gomme arabique disponible sur le marché est souvent insuffisante, que ce soit dû aux variations climatiques dans les lieux de production ou des échanges commerciaux difficiles avec les pays producteurs ; en outre, la qualité de gomme arabique commercialisée est irrégulière, ce qui implique des modifications fréquentes et coûteuses des conditions de fabrication des microcapsules.

Les microcapsules, selon l'invention, sont constituées de gélatine et de polysaccharide anionique de faible masse moléculaire, c'est-à-dire dont la viscosité des solutions aqueuses à 15 g/l (1,5 %), mesurée à 75°C avec un viscosimètre Brookfield, est comprise entre 1 et 20 mPa.s environ.

Les polysaccharides utilisables dans le cadre de la présente invention ne se trouvent en général pas dans la nature ; ils sont obtenus par dépolymérisation, par une méthode physique, chimique ou enzymatique, des polysaccharides anioniques naturels hydrosolubles ou rendus hydrosolubles. Ces polysaccharides peuvent être choisis parmi les alginates, extraits d'algues brunes, les carraghénanes, de type lambda, iota, ou kappa extraits d'algues rouges, les pectines extraites des citrons, des pommes ou des betteraves, les pectates, qui résultent de la déméthylation des pectines, ou encore les carboxyméthylcelluloses, les carboxyméthylguars ou les carboxyméthylamidons.

On préfère, tout particulièrement, les carraghénanes, notamment parce que la coacervation avec une gélatine de type A a lieu sans ajustement du pH du milieu, et parce que le durcissement des parois des microcapsules lors du refroidissement de la suspension est rapide. Certains de ces polysaccharides dépolymérisés sont commercialisés.

Les alginates, les carraghénanes et les pectines faiblement méthoxylées (LM) peuvent être dépolymérisés par voie chimique, par hydrolyse en milieu acide, tandis que les pectines fortement méthoxylées (HM) seront dépolymérisées par hydrolyse en milieu basique ; on peut aussi effectuer la dépolymérisation des pectines par action d'une pectinase en milieu aqueux. Ces conditions de dépolymérisation sont classiques, bien connues de l'homme de l'art, qui pourra sans difficulté choisir celles qui permettront d'obtenir un polysaccharide dépolymérisé suffisamment homogène dont la solution aqueuse aura une viscosité, convenant à la préparation des microcapsules de l'invention, ce qui dépend de la nature du produit de départ. Une autre méthode de dépolymérisation consiste à irradier une poudre de polysaccharide, par exemple comme décrit dans le brevet brésilien, déposé le 17 décembre 1979 sous le n° 79 08661 au nom de HELIO DA IGREJEA et publié le 30 juin 1981.

Les carboxyméthylcelluloses de faible masse moléculaire peuvent être obtenues soit par dépolymérisation de produits de haute masse moléculaire, soit par une réaction de polymérisation contrôlée à partir des oses par un procédé connu en lui-même.

Les microcapsules de l'invention sont préparées avec des polysaccharides de faible masse moléculaire, définie par la viscosité de leurs solutions aqueuses à 15 g/l, mesurée à 75°C avec un viscosimètre rotatif Brookfield de type LVT équipé d'un mobile UL à vitesse de rotation de 6 tours/minute. La viscosité convenable dépend du polysaccharide considéré. Elle est, de préférence :
- pour les alginates, comprise entre 1 et 4 mPa.s environ et de préférence entre 1 et 3 mPa.s environ ;
- pour les carraghénanes, comprise entre 1 et 10 mPa.s environ et de préférence entre 2 et 8 mPa.s environ ;
- pour les pectines, comprise entre 1 et 20 mPa.s environ et de préférence entre 1 et 15 mPa.s environ ;
- pour les carboxyméthylcelluloses sodiques, comprise entre 1 et 15 mPa.s environ et de préférence entre 1 et 10mPa.s environ ;
- et, pour les pectates, comprise entre 1 et 15 mPa.s environ.

La gélatine, mise en oeuvre dans l'invention, peut être obtenue par hydrolyse acide ou basique du collagène d'os, de peaux ou de tendons d'ovins, de porcins ou de bovins ; elle a une force en gelée de 50 à 300 blooms, et de préférence de 90 à 250 blooms, mesurée par la méthode décrite dans le British Standard.

Les microcapsules sont formées à partir d'un mélange contenant 1/3 à 1/40 en poids environ, et de préférence 1/4 à 1/20, par rapport au poids de gélatine, d'un ou de plusieurs polysaccharides dépolymérisés.

Les solutions de gélatine contiennent de 2,5 g à 150 g de gélatine par kg et de préférence de 10 à 100 g/kg. Les solutions de polysaccharide dépolymérisé contiennent de 0,5 g à 50 g de polysaccharide par kg et de préférence de 1 g à 30 g/kg.

La proportion préférée du polysaccharide varie avec la nature de celui-ci ; elle sera par exemple de 1/5 à 1/4 pour les alginates, de 1/3 à 1/4 pour les carboxyméthylcelluloses, de 1/4 à 1/7 pour les pectines, de 1/6 à 1/10 pour les kappa carraghénanes, de 1/9 à 1/14 pour les lambda carraghénanes et de 1/10 à 1/16 pour les iota carraghénanes.

Le mélange qui est coacervé contient donc une forte proportion de gélatine, et sera par conséquent plus facile à gélifier et à durcir que celui utilisé pour les microcapsules de l'art antérieur à base de gomme arabique, dans lequel les deux polymères étaient en proportions équivalentes.

Les proportions relatives de gélatine et de polysaccharide dans la paroi des microcapsules isolées peuvent différer de celles dans le mélange de départ ; en général, la proportion de polysaccharide est inférieure à celle introduite dans le milieu de coacervation.

Un autre objet de l'invention est le procédé de préparation des microcapsules.

Il consiste essentiellement à introduire, sous agitation, la solution aqueuse de polysaccharide, à une température supérieure à celle de gélification, dans une solution aqueuse de gélatine maintenue à une température supérieure à sa température de gélification, entre 30°C et 80°C environ, le produit liquide ou solide à enrober étant préalablement émulsionné ou mis en suspension dans l'une ou l'autre des solutions aqueuses de polymère.

Le pH du milieu est ensuite amené, si nécessaire, à une valeur inférieure au point isoélectrique de la gélatine, laquelle doit être sous forme cationique pour former un coacervat avec le polysaccharide anionique. Par exemple, pour la gélatine de type A, préparée par hydrolyse acide du collagène, le pH sera compris entre 3,5 et 6,0 et de préférence entre 4,0 et 5,2 par addition d'un acide ou d'une base minérale ou organique, tels que HCl, $H_2SO_4$, $CH_3COOH$ ou NaOH et KOH.

On peut aussi mélanger les deux solutions chaudes de polymères avant d'y introduire le produit liquide ou solide à enrober, surtout dans le cas où il n'y a pas une coacervation spontanée du mélange des polymères.

Les polysaccharides dépolymérisés sont dissous dans l'eau par une simple agitation mécanique, ce qui est un autre avantage de l'invention puisque la dissolution de la gomme arabique ou celle des polysaccharides naturels suppose la mise en oeuvre d'agitation violente par turbine et même l'utilisation d'un tiers solvant, comme l'alcool éthylique, pour les pectines.

Le produit à enrober est introduit dans le milieu en général à raison de 1/6 à 1/20 en volume, en fonction de sa densité. Lorsque c'est un solide pulvérulent, la quantité introduite dépend aussi de la mouillabilité du solide.

On refroidit ensuite le milieu, toujours sous agitation, à une température inférieure ou égale au point de gélification de la gélatine utilisée pour solidifier les parois des microcapsules. Ces parois sont en général trop fragiles pour être isolées, même après quelque temps de durcissement à cette température, et on ajoute à la suspension, 1 à 5 h après le refroidissement du milieu, une solution aqueuse d'un agent de réticulation de la gélatine, tel que le formaldéhyde, le glyoxal ou le glutaraldéhyde ou d'un agent tannant naturel, tel que l'acide tannique pour compléter le durcissement des parois, en application d'un principe classique.

Les concentrations des solutions de formaldéhyde et de glutaraldéhyde ajoutées sont, en général, celles des solutions commerciales, soit respectivement 37 % (p/v) et 25 % (p/v) ; pour l'acide tannique elle sera de 0,5 à 5 %. On mettra d'autant plus d'agent réticulant que la capsule à obtenir devra être plus solide et moins soluble. Ce durcissement peut se faire au pH de coacervation mais, particulièrement lorsque l'on utilise le formaldéhyde, on préfère l'effectuer à un pH basique, de 10 environ. Sa durée est fonction de l'agent de réticulation, de sa concentration, de la taille des particules ; elle est en général comprise entre quelques minutes et une heure.

Après la fin de la réticulation, on arrête l'agitation et sépare les microcapsules de la solution par un moyen classique tel qu'une décantation, une filtration ou une centrifugation. La masse obtenue est égouttée puis lavée à l'eau. Le séchage de ces microcapsules peut être effectué en courant d'air, de préférence en lit fluidisé ou par atomisation. On préfère effectuer le séchage en présence d'un additif de séchage pulvérulent tel que la silice, l'amidon ou l'alumine. Pour cela, les microcapsules lavées sont mises en suspension dans un peu d'eau, en présence de l'additif de séchage ; on sépare ensuite l'eau par un moyen classique avant de sécher la masse pâteuse.

Ce séchage peut être effectué par circulation d'air dans un séchoir à plateau ou en tunnel, dans une étuve, en lit fluidisé ou par lyophilisation. On sépare ensuite l'additif de séchage des microcapsules de l'invention par tamisage.

Par ce procédé, on obtient des microcapsules de diamètre moyen compris entre 50 µm et 700 µm, qui contiennent de 40 à 90 % en poids de produit encapsulé ; le diamètre moyen dépendra de la taille des particules à encapsuler et par conséquent de l'émulsification dans le cas d'encapsulation des liquides.

On peut enrober un grand nombre de produits par le procédé de l'invention ; il faut évidemment que ceux-ci ne soient pas solubles dans l'eau, ou miscibles à l'eau pour les liquides, et que la paroi liquide, constituée du mélange des deux polymères, mouille le produit à encapsuler. Parmi les produits encapsulables, on peut citer les adhésifs, et particulièrement les adhésifs à deux composants, les lubrifiants, les colorants, les produits cosmétiques comme les parfums, les huiles, les colorants, les produits alimentaires tels qu'arômes et épices, les produits pharmaceutiques, notamment ceux ayant un goût désagréable, organiques ou minéraux, comme le sulfate de baryum, les engrais, les pesticides et les insecticides qui seront relargués plus lentement dans leur milieu.

## Exemple 1

### Microencapsulation d'huile avec de la gélatine et un alginate dépolymérisé.

Dans un bécher de 100 ml, on introduit 5 g de gélatine de type A (c'est-à-dire résultant d'une extraction acide) de point isoélectrique 9,1, ayant une force en gelée de 245 blooms, puis 45 ml d'eau déminéralisée ; après 30 min de gonflement, la gélatine est fondue par chauffage à 50°C au bain-marie ; à cette température, on introduit 40 ml d'huile de tournesol et on l'émulsionne par agitation du milieu avec un agitateur magnétique, durant 10 minutes.

On introduit alors 300 ml d'une solution, à 50°C, d'alginate dépolymérisé, préparée à partir de 1,23 g de poudre de polysaccharide dans 298,77 g d'eau déminéralisée.

L'alginate est la Satialgine®, comercialisée par la Société Mero Rousselot Satia (France) ; la viscosité de la solution aqueuse à 1,5 %, mesurée comme décrit ci-dessus, est de 3 mPa.s.

Après quelques minutes d'agitation, on ajoute une solution aqueuse d'acide acétique 1N jusqu'à obtenir un milieu à pH 4,6.

L'agitation à 50°C est maintenue 15 minutes puis on abaisse progressivement, en 1 heure, la température du milieu à 10°C.

Après 2 heures à cette température, pendant lesquelles les parois des capsules néoformées se gélifient, on ajoute à la suspension, sous agitation, 4 ml de solution aqueuse de formaldéhyde à 37 % (p/v) et une solution aqueuse de NaOH 10N jusqu'à pH 10.

Au bout de 30 minutes, les microcapsules dures sont séparées du milieu par filtration sur tamis puis lavées à l'eau à 15°C environ.

On mélange ensuite la masse humide dans 100 ml d'eau avec 10 g de silice pulvérulente et on filtre pour isoler un gâteau contenant les microcapsules et la silice hydratée.

Ce gâteau est introduit à température ambiante dans la cuve d'un sécheur à lit fluidisé, dans lequel circule de l'air. Lorsqu'une poudre sèche, coulant librement, s'est formée, on l'isole et la tamise pour éliminer la silice.

On obtient ainsi 45 g de microcapsules, non collantes, qui sont étanches et stables au stockage et contiennent 40 ml d'huile ; la paroi comporte 12 % d'alginate par rapport au poids de gélatine.

## Exemple 2

### Microencapsulation d'huile avec de la gélatine et un carraghénane dépolymérisé.

En suivant le mode opératoire décrit à l'exemple 1, on prépare une émulsion à 50°C de 50 ml de solution à 5 % (p/v) de gélatine de type A, de point isoélectrique 8,9 et de force en gelée 225 blooms, avec 50 ml d'huile essentielle de bergamote, ainsi que 250 ml d'une solution à 0,18 % (p/v) de carraghénane dépolymérisé, commercialisé par la Société Mero Rousselot Satia sous la marque Stabilgum® (viscosité de la solution aqueuse mesurée dans les conditions ci-dessus : 6 mPa.s).

A 50°C, on mélange l'émulsion et la solution et, après quelques minutes, on introduit une solution aqueuse de HCl 1N, jusqu'à ce que le pH du milieu soit 4,8.

Après 15 minutes d'agitation, on abaisse la température jusqu'à 10°C en 2 heures, et continue l'agitation pendant 2 heures.

On introduit alors 3 ml de solution aqueuse de formaldéhyde à 37 % et une solution aqueuse de NaOH 10N jusqu'à pH 10. Après 30 minutes d'agitation, on centrifuge 2000 tours/minute pour séparer les microcapsules ; celles-ci sont lavées et traitées par la silice comme dans l'exemple précédent. Le séchage est cette fois réalisé en étuve ventilée à plateaux.

On obtient ainsi 54 g de microcapsules, de diamètre inférieur à 700 μm, contenant 50 ml d'huile. Leur paroi contient 8,2 % de carraghénane.

### Exemple 3

Microcapsulation d'huile avec de la gélatine et une pectine dépolymérisée.

On émulsionne à 40°C, en suivant le mode opératoire décrit dans l'exemple précédent, 50 ml d'une solution à 10 % (p/v) de gélatine et 40 ml d'huile d'amande douce.

On ajoute à cette émulsion 30 ml d'une solution aqueuse 0,4 % (p/v) de pectine dépolymérisée, commercialisée sous la dénomination Brun G par Mero Rousselot Satia (viscosité : 19 mPa.s).

On effectue ensuite les différentes étapes de coacervation, réticulation, isolement et séchage comme décrit à l'exemple 1 pour obtenir 45 g de microcapsules contenant 40 ml d'huile. Leur paroi comprend 15 % de pectine.

### Exemple 4

Microcapsulation avec de la gélatine et une carboxyméthylcellulose pour obtenir une colle à deux composants.

On émulsionne à 60°C, d'une part, 180 ml d'une solution aqueuse à 33 % (p/v) de gélatine de type A, de point isoélectrique 8,5 et de force en gelée 180 blooms, avec 50 ml de composé A d'une colle époxy à deux composants et, d'autre part, 180 ml de la même solution de gélatine avec 50 ml du composé B de la même colle.

On ajoute à 60°C, à chacune de ces deux émulsions, 80 ml d'une solution à 1 % (p/v) de carboxyméthylcellulose de faible masse moléculaire, commercialisée par Wolff Walsrode AG, sous la marque Walocel CRT 30 G (viscosité : 7 mPa.s). Après quelques minutes d'agitation, on abaisse le pH du milieu à 4,5 par addition d'une solution aqueuse d'acide acétique 1N. 10 minutes après, la température est abaissée jusqu'à 10°C, et on laisse le mélange sous agitation pendant 4 heures à cette température ; on introduit alors 1 ml d'une solution aqueuse de glutaraldéhyde à 25 % (p/v) et sépare les microcapsules durcies au bout de 30 minutes.

Après isolement, lavage et séchage, on obtient deux ensembles de microcapsules dont l'un contient le composé A d'une colle époxy et, l'autre, le composé B. Leur paroi comprend 16 % de carboxyméthylcellulose. Ces ensembles peuvent être mélangés sans que les deux composants réagissent. La colle ne se formera que lorsqu'on appliquera une pression sur le mélange, pour expulser les composés A et B des microcapsules. Le mélange est stable pendant plus de 2 ans.

### Exemple 5

Microencapsulation d'un solide avec de la gélatine et un alginate dépolymérisé.

On verse 45 ml d'eau déminéralisée sur 5 g de gélatine de type A de point isoélectrique 9,1 ayant une force en gelée de 225 blooms. Après 30 minutes durant lesquelles la gélatine gonfle, elle est fondue par chauffage à 40°C. A cette température, on introduit 35 g d'une poudre de benzodiazépine de granulométrie moyenne 120 μm et réalise une suspension par agitation pendant 10 minutes avec un agitateur mécanique tournant à 200 tours/minute. On introduit alors, à 50°C, 300 ml d'une solution d'alginate dépolymérisé, préparée avec 1,23 g d'alginate pour 100 g d'eau déminéralisée. L'alginate est une Satialgine®, commercialisée par la Société Mero Rousselot Satia (France) ; la viscosité de la solution aqueuse à 1,5 % (p/v), mesurée dans les conditions précédemment définies, est de 5 mPa.s.

Après quelques minutes d'agitation, on ajoute une solution aqueuse d'acide acétique 1N jusqu'à pH 4,4. On continue d'agiter à 50°C pendant 15 minutes puis la température du milieu est abaissée en 1 heure à 10°C.

Après 3 heures d'agitation à cette température, on agite 15 ml d'une solution aqueuse à 1 % d'acide tannique et après 15 minutes on sépare les microcapsules par tamisage. Elles sont lavées avec l'eau à 15°C environ, puis la masse humide est mélangée avec 15 g de silice pulvérulente dans 100 ml d'eau, avant de filtrer pour isoler un gâteau qui est séché comme dans l'exemple 2. On obtient ainsi 40 g de microcapsules sèches, en poudre, s'écoulant librement et contenant 35 g de principe actif. Leur paroi contient 11 % d'alginate.

### Exemple 6

Microencapsulation d'une huile avec de la gélatine et un carraghénane dépolymérisé.

On introduit dans un bécher de 100 ml 2,5 g de gélatine de type A, de point isoélectrique 9,1 et de force en gelée 240 blooms ainsi que 47,5 ml d'eau déminéralisée ; la gélatine est laissée gonfler pendant 30 minutes puis est fondue au bain-marie 50°C. On ajoute alors 50 ml d'huile essentielle de céleri et émulsionne, sous simple agitation magnétique, le mélange avant d'introduire à 50°C 250 ml d'une solution aqueuse contenant 2,4 g de iota carraghénane dont la viscosité de la solution aqueuse, mesurée comme indiqué précédemment, est de 6 mPa.s.

La coacervation se produit instantanément dès que le pH 4,8 est atteint ; on abaisse au bout de 10 minutes environ la température à 10°C en 1 heure et laisse la suspension sous agitation 1/2 heure à cette température.

On introduit ensuite dans le milieu 20 ml d'une solution aqueuse d'acide tannique à 5 %. Après 15 minutes, les microcapsules sont séparées sur un filtre et lavées à l'eau froide. Elles sont alors mélangées avec 10 g de silice pulvérulente en suspension dans 100 ml d'eau ; la phase aqueuse est séparée par filtration et la pâte séchée dans un lit d'air fluidisé à température ambiante. La poudre sèche est tamisée sur un tamis de maille 20 μm pour séparer la silice. On obtient ainsi 47,5 g de microcapsules, contenant 33,25 g d'huile ; leur paroi comprend 7,9 % de carraghénane.

### Exemple 7

Microencapsulation d'une huile essentielle de citron avec de la gélatine et un carraghénane dépolymérisé.

On prépare une solution aqueuse de la gélatine comme l'exemple 6 mais de concentration 10 % et l'émulsionne à 30°C avec 50 ml d'huile essentielle de citron.

A cette température, on introduit dans l'émulsion 200 ml d'une solution aqueuse contenant 0,5 g de carraghénane lambda dont la viscosité est 7 mPa.s. Le pH du mélange est 5 et la coacervation se produit spontanément. Après quelques minutes, on abaisse, en 1 heure environ, la température à 5°C et on abandonne sous agitation pendant 1 heure avant d'introduire 20 ml d'une solution aqueuse à 5 % d'acide tannique. Après 15 minutes, on sépare les microcapsules de la phase aqueuse par centrifugation et on les sèche comme à l'exemple 6. On obtient ainsi 45 g de microcapsules contenant 29,7 g d'huile dont les parois comprennent 9,3 % de carraghénane.

### Exemple 8

Microencapsulation d'une huile essentielle de pin avec de la gélatine et un carraghénane dépolymérisé.

On prépare une solution aqueuse à 5 % de gélatine de type A, de point isoélectrique 8,8 et de force en gelée 185 blooms. On émulsionne à 50°C 50 ml de cette solution avec 30 ml d'huile essentielle de pin, et on introduit dans l'émulsion 250 ml d'une solution aqueuse contenant 0,36 g de kappa carraghénane. Le pH du milieu est de 5 et la coacervation est immédiate. Après 15 minutes environ, on abaisse la température à 10°C en 1 heure et abandonne sous agitation 1 heure avant d'introduire 0,5 ml de solution de NaOH 10N pour atteindre un pH 10. Après 15 minutes les microcapsules sont isolées et séchées comme décrit l'exemple 6. On obtient 28 g de microcapsules contenant 20 g d'essence, qui libèrent l'essence de pin en milieu aqueux vers 70°C ; leurs parois contiennent 11,8 g de kappa carraghénane.

## Revendications

1. Procédé de préparation de microcapsules contenant un liquide hydrophobe ou un solide insoluble dans l'eau, par coacervation complexe d'un mélange de gélatine et de polysaccharide anionique hydrosoluble de faible masse moléculaire.

2. Procédé de préparation selon la revendication 1, caractérisé en ce que le polysaccharide de faible masse moléculaire est un polysaccharide dépolymérisé par voie chimique, choisi parmi les alginates, les carraghénanes, les pectines et les pectates, les carboxyméthylcelluloses, les carboxyméthylguars et les carboxyméthylamidons.

3. Procédé selon l'une des revendications 1 ou 2, caractérisé en ce que la solution de gélatine de départ contient de 2,5 à 150 g de gélatine par kg.

4. Procédé selon l'une quelconque des revendications 1 à 3, caractérisé en ce que le polysaccharide est un alginate dont la viscosité est comprise entre 1 et 4 mPa.s.

5. Procédé selon la revendication 4, caractérisé en ce que le mélange de coacervation comprend 1/5 à 1/4 en poids d'alginate par rapport à la gélatine.

6. Procédé selon l'une quelconque des revendications 1 à 3, caractérisé en ce que le polysaccharide est un carraghénane dont la viscosité est comprise entre 1 et 10 mPa.s.

7. Procédé selon la revendication 6, caractérisé en ce que le mélange de coacervation comprend 1/6 à 1/10 en poids de kappa carraghénane par rapport à la gélatine.

8. Procédé selon la revendication 6, caractérisé en ce que le mélange de coacervation comprend de 1/9 à 1/14 en poids de lambda carraghénane par rapport à la gélatine.

9. Procédé selon la revendication 6, caractérisé en ce que le mélange de coacervation comprend de 1/10 à 1/16 en poids de iota carraghénane par rapport au poids de gélatine.

10. Procédé selon l'une quelconque des revendications 1 à 3, caractérisé en ce que le polysaccharide est une pectine dont la viscosité est comprise entre 1 et 20 mPa.s.

11. Procédé selon la revendication 10, caractérisé en ce que le mélange de coacervation comprend de 1/4 à 1/7 en poids de pectines par rapport à la gélatine.

12. Procédé selon l'une quelconque des revendications 1 à 3, caractérisé en ce que le polysaccharide est un pectate dont la viscosité est comprise entre 1 et 5 mPa.s.

13. Procédé selon l'une quelconque des revendications 1 à 3, caractérisé en ce que le polysaccharide est une carboxyméthylcellulose dont la viscosité est comprise entre 1 et 15 mPa.s.

14. Procédé selon la revendication 13, caractérisé en ce que le mélange de coacervation comprend de 1/3 à 1/4 en poids de carboxyméthylcellulose par rapport au poids de gélatine.

15. Procédé selon l'une quelconque des revendications 1 à 14, caractérisé en ce que les microcapsules formées par coacervation sont ensuite réticulées par action d'un agent durcissant la gélatine.

16. Procédé selon l'une quelconque des revendications 1 à 15, caractérisé en ce que les microcapsules sont séchées en présence d'un additif choisi parmi la silice, l'amidon et l'alumine puis séparées de l'additif par tamisage.

17. Microcapsules de gélatine et polysaccharide de faible masse moléculaire, préparées par le procédé de l'une quelconque des revendications 1 à 16.

18. Microcapsules selon la revendication 17, caracterisées en ce qu'elles se présentent sous forme sèche.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 378 561 (KANZAKI PAPER) <br> * Page 4, lignes 29-34; page 15, lignes 2-38; page 16, lignes 1-5; page 7, lignes 7-10; page 32, tableau IV * <br> --- | 1,2,13, 15 | B 01 D 13/02 <br> A 61 K 9/50 |
| A | FR-A-1 599 886 (MELLE-BEZONS) <br> * Page 2, lignes 33-35 * <br> --- | 1,2 | |
| A | EP-A-0 101 891 (G.D. SEARLE & CO.) <br> * Page 12, revendication 1 * <br> ----- | 1 | |

DOMAINES TECHNIQUES RECHERCHES (Int. Cl.4)

B 01 D
A 61 K

Le présent rapport a été établi pour toutes les revendications

| Lieu de la recherche | Date d'achèvement de la recherche | Examinateur |
|---|---|---|
| LA HAYE | 01-03-1988 | KERRES P.M.G. |